(19) Europäisches Patentamt
European Patent Office
Office européen des brevets

(11) Publication number: **0 021 293**
**B1**

(12) **EUROPEAN PATENT SPECIFICATION**

(45) Date of publication of patent specification: **07.09.83**

(51) Int. Cl.³: **C 07 H 19/06, A 61 K 31/70**

(21) Application number: **80103326.7**

(22) Date of filing: **13.06.80**

(54) **4-Substituted thio-1-beta-D-ribofuranosylpyrazolo(3,4-D) pyrimidines, processes for their preparation, pharmaceutical formulations and medical uses.**

(30) Priority: **14.06.79 GB 7920698**
**14.06.79 GB 7920699**
**14.06.79 GB 7920700**

(43) Date of publication of application:
**07.01.81 Bulletin 81/1**

(45) Publication of the grant of the patent:
**07.09.83 Bulletin 83/36**

(84) Designated Contracting States:
**BE CH DE FR GB LI NL SE**

(56) References cited:
**JOURNAL OF HETEROCYCLIC CHEMISTRY vol. 14, no. 3, May 1977, page 483 J. L. G. MONTERO et al.: "Pyrazolopyrimidine Nucleosides. Part VIII (1) The Synthesis of Certain 4-Substituted Pyrazolo (3, 4-d) pyrimidine Nucleosides"**

(73) Proprietor: **THE WELLCOME FOUNDATION LIMITED**
**183-193 Euston Road**
**London NW1 2BP (GB)**

(72) Inventor: **Rideout, Janet Elizabeth**
**3101 Morningside Drive**
**Raleigh North Carolina 27607 (US)**
Inventor: **Krenitsky, Thomas Anthony**
**106 Laurel Hill Road**
**Chapel Hill North Carolina 27514 (US)**
Inventor: **Elion, Gertrude Belle**
**Oxford Apartments 1, Banbury Lane**
**Chapel Hill North Carolina 27514 (US)**

(74) Representative: **Berg, Wilhelm, Dr. et al,**
**Dr. Berg, Dipl.-Ing. Stapf, Dipl.-Ing. Schwabe, Dr. Dr. Sandmair Mauerkircherstrasse 45**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

4-Substituted thio-1-beta-D-ribofuranosylpyrazolo (3,4-D) pyrimidines, processes for their preparations, pharmaceutical formulations and medical uses.

The present invention relates to 4-(substituted)thio-1-$\beta$-D-ribofuranosylpyrazolo[3,4-*d*]pyrimidine derivaties which are useful as antiprotozoal agents, especially for treating or preventing coccidiosis.

Coccidiosis is caused by protozoa of the genus *Eimeria*, which infect susceptible hosts by contact with faeces of diseased animals. It is therefore particularly damaging when animals are kept in close contact, and is thus the most important disease of poultry. Various therapeutic and prophylactic agents are known for combatting coccidiosis and are used with differing degrees of success. These are usually administrated throughout the life of animals and there is consequently a risk of the protozoa developing resistance to one or more of these agents.

4-Methylthio-1-$\beta$-D-ribofuranosyl pyrazolo[3,4-*d*]pyrimidine and close analogues were prepared as potential anticancer agents, (J. L. G. Montero *et al., J. Hetero Chem., 14,* 483, (1977) R. P. Panzica, *et al.,* R. E. Harman, R. K. Robinsons and L. B. Townsend (Eds.); Chemistry and Biology of Nucleosides and Nucleotides, Academic Press, New York; (1978), 121—134) but no other type of biological activity has been disclosed.

The 4-methylthiopyrazolo[3,4-*d*]pyrimidine riboside has now been tested against coccidia and whilst it has good *in vitro* activity it was found to be highly toxic, causing unacceptable fatalities in chickens.

It has now been found that 4-(substituted)thiopyrazolo[3,4-*d*]pyrimidine ribosides, in which the substituent on the sulphur atom is an alkenyl or alkyl group larger than a methyl group, are also active against protozoa of the genus *Eimeria*. In contrast with the known 4-methylthio derivative, these compounds have surprisingly low toxicity towards the host animal and are therefore suitable for treating or preventing coccidiosis in poultry.

According to the present invention therefore there is provided a compound of formula (I)

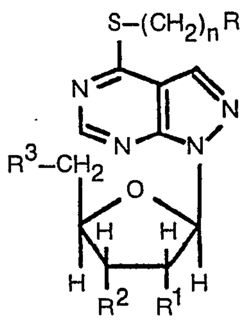

(I)

wherein n is an integer of 1 to 6 and R is a methyl, $(C_{1-4})$ alkoxy or $(C_{1-4})$alkylthio group or a phenoxy or phenylthio group or an unsubstituted or monosubstituted phenyl group, or when n has the value 1, a group $—(CH{=}CH)_mR^5$ wherein m has the value 1 or 2 or a group $—C{\equiv}C—R^5$ wherein $R^5$ is an unsubstituted or mono-, di-, or tri-substituted phenyl group, substituents for either of the aforementioned phenyl groups being selected from halogen atoms and $(C_{1-4})$alkyl, $(C_{1-4})$alkoxy, trifluoromethyl, benzyloxy, phenoxy, amino, mono- or di-$(C_{1-4})$alkylamino and hydroxyl groups, and either $R^1$, $R^2$ and $R^3$ are the same and are hydroxyl or acyloxy groups —O—CO—$R^4$ wherein $R^4$ is a hydrogen atom or a $(C_{1-4})$alkyl group or a substituted or unsubstituted phenyl group or $R^1$ and $R^2$ are hydroxyl or acyloxy groups as hereinbefore defined and $R^3$ is a phosphate group, or a salt thereof.

When $R^4$ is present as a phenyl group it may be optionally substituted with one or more of the substituents commonly known in the art and used as substituents for benzoyl esters of nucleosides and nucleotides, such as amino, hydroxyl, nitro, $(C_{1-4})$alkyl and $(C_{1-4})$alkoxyl groups and halogen atoms.

As used herein the terms "$(C_{1-4})$alkyl group" and "$(C_{1-4})$alkoxy group" refer to such groups having from 1 to 4 carbon atoms.

If $R^4$ represents the salt of a phosphate group it is preferred that it is a pharmaceutically acceptable salt, such as the sodium or potassium salt in a mono or dibasic form.

In compounds of formula (I) where R is a methyl group it is preferred that n has the value 1 to 5. When n has the value 1 and R is a gorup $—(CH{=}CH)_mR^5$, it is preferred that m has the value 1. When R, in a compound of formula (I), is a phenyl group it is preferred that n has the value 1 to 3.

Compounds are particularly preferred when they embody two or more of the preferred features outlined above.

The most preferred compounds are 4-cinnamylthio- and 4-ethylthio-1-$\beta$-D-ribofuranosyl-pyrazolo[3,4-*d*]pyrimidines, their phosphate esters and salts thereof.

Compounds of formula (I) may be prepared either by modification of the 4-substituent of a 1 - $\beta$ - D - ribofuranosylpyrazolo[3,4-*d*]pyrimidine (the precursor), or by linking the ribose moiety to a pyrazolo[3,4-*d*]pyrimidine derivative already bearing the correct atom or group at the 4-position.

According to a second aspect of the present invention there is therefore provided a process for producing compounds of formula (I) comprising the reaction between the precursor, a 4 - (substituted) - 1 - $\beta$ - D - ribofuranosylpyrazolo[3,4-*d*]pyrimidine derivative and a compound $R(CH_2)_nX$ wherein n and R are as hereinbefore defined and X is a halogen atom and the 4-substituent of the precursor is a thio group and the reaction is performed in the presence of an organic or inorganic base or a basic resin in an aqueous, a lower alcoholic or an aprotic solvent.

As used herein in relation to the precursor the term "4-(substituted) - 1 - $\beta$ - D - ribofuranosyl pyrazolo[3,4-*d*]pyrimidine derivative" includes such organic and phosphate esters, and salts of the latter, as are appropriate to the final product of the process.

In the above the halogen atom may be a chlorine, bromine or iodine atom. The base used in this method may be an alkali or alkaline earth metal hydroxide or alkoxide, quaternary ammonium hydroxide, hydrogen carbonate or carbonate, or a basic resin such as Dowex 1—X8 (bicarbonate) (Dowex is a Registered Trade Mark) supplied by Bio-Rad Laboratories California, USA. The solvent may be water, a lower alcohol, such as methanol or ethanol or an aprotic solvent such as N,N-dimethylformamide, dimethylsulphoxide or hexamethylphosphoric triamide, although N,N-dimethylformamide is preferred.

Whenever the compound of formula (I) is required to carry acyloxy groups for $R^1$, $R^2$, and $R^3$, a corresponding starting compound having hydroxy groups in these positions is reacted with acylating agents such as acetic anhydride or benzoyl chloride according to conventional methods. Acylation may be effected before or after other synthetic steps except that when enzymatic or microbiological processes are top be used for ribosidation the acylation must be performed after ribosidation.

When $R^3$ of the desired compound of formula (I) is to be a phosphate group, this may be introduced into the corresponding compound having a hydroxyl group in that position by phosphorylation using traditional phosphorylating agents such as trialkyl phosphates, eg triethyl phosphate, or with a phosphorus oxyhalide such as phosphoryl chloride. When this technique is used it is advantageous to block the 2′ and 3′ positions of the ribose moiety either by blocking only these two positions by using appropriate conditions or by blocking the 2′, 3′ and 5′ positions and then selectively deblocking the 5′ position. The latter course may be facilitated by first blocking the 5′ position with a bulky group, such as a trityl group or a *t*-butyldimethylsilyl group, then blocking the 2′ and 3′ positions by conventional means, and finally deblocking the 5′ position. After phosphorylation the 2′ and 3′ positions are then deblocked to afford the required compound.

Rather than block the 2′ and 3′ positions as described baove, it is preferred to use phosphoryl chloride in the presence of a trialkylphosphate (preferably triethyl phosphate) and a trace of water at a temperature of about 0°C or below. This forms the 5′-phosphoro dichloride which is then hydrolysed to the 5′-phosphate upon treatment with water at slightly basic pH.

Salts of phosphate-substituted compounds of formula (I) are obtained by conventional reactions between the phosphate derivative and an appropriate base in aqueous media.

The precursors for use in the preparation of compounds of formula (I) may be well known compounds such as 4-hydroxy-, 4-thio- or 4 - methylthio - 1 - $\beta$ - D - ribofuranosylpyrazolo[3,4-*d*]pyrimidine derivatives, or may be obtained therefrom by conventional techniques. These ribosides may have been prepared from the corresponding free pyrazolo[3,4-*d*]pyrimidine bases by ribosidation.

Chemical processes may be employed in which the donor system comprises a reactive ribose derivative such a a 1-chlororibose derivative, the reaction being performed in an appropriate solvent system such as an aprotic solvent, e.g. dimethylformamide or acetonitrile. However, it is preferred that enzymatic processes be used.

Such enzymatic processes include using phosphorylase type enzymes in a manner known in the art: see for instance T. A. Krenitsky, G. B. Elion, R. A. Strelitz, G. H. Hitchings, *J. Biol. Chem., 242*, 2675—2682, (1967); UK Patent Application No. 45668/77; see also European Patent Application No. 78 101 295.0, Publication No. 0 002 192, in which case the riboside donor system consists of appropriate purine and/or pyrimidine-1-$\beta$-D-ribosides and/or ribose-1-phosphate and the enzyme or enzymes.

Alternatively the ribosidation may be accomplished by microbiological processes such as that disclosed in German Offenlegungsschrift No. 2 209 078 wherein the riboside donor system comprises bacteria of the genera *Brevibacterium, Arthrobacter, Corynebacterium or Micrococcus* and the culture medium which includes glucose.

The 4-thio substituted precursor may be obtained from the acylated 4-halogeno-pyrazolo[3,4-*d*]-pyrimidine riboside by treatment of the latter with thiourea or sodium hydrosulphide. The 4-halogeno precursor can be derived by treating an acylated derivative of 4-hydroxy-pyrazolo[3,4-*d*]pyrimidine riboside with a phosphoryl halide, the corresponding Villsmeier reaction or other known halogenating agents.

The 4-alkylthio- and 4-aralkylthio substituted precursors may be derived from other compounds of this class, from the 4-halogeno precursor or from the 4-thio precursor by the process for preparing compounds of formula (I), *mutatis mutandis*.

Simple reagents of the formula $R(CH_2)_nX$ for use in producing compounds of formula (1) may be available commercially (e.g. from Aldrich Chemical Co., Milwaukee, Wisconsin, USA). However all these

reagents $R(CH_2)_nX$ may be produced by methods well known in the art. Those reagents wherein R is a phenoxy, phenylthio, alkoxy or alkylthio group are produced by the following methods from either the appropriate ω-halogenoalkyl alcohol or α,ω-dihalogeno alkane.

The ω-halogenated alcohols are generated by reduction of the corresponding ω-halogenoalkyl carboxylic acid, chloride or ester using reducing agents such as lithium aluminium hydride or sodium borohydride or by catalytic hydrogenation using a catalyst such as platinum oxide. The ω-halogenoalkyl alcohol is then reacted with the alkoxide, phenoxide, thiolate or phenylthiolate corresponding to the R moiety, (which is generated by the action of an alkali metal or its hydride, carbonate or methoxide, on the appropriate alcohol or thiol) in an aprotic solvent such as N,N-dimethylformamide, diglyme, ether or dimethylsulphoxide or in the alcohol or thiol corresponding to R, at a temperature between 20°C and 150°C, preferably up to 100°C. The ω-hydroxyether or ω-hydroxythioether so formed is then halogenated by methods known in the art to afford the required reagent $R(CH_2)_nX$.

Alternatively an α,ω-dihalogenated alkane is added, in greater than three fold excess, to a solution of the metal alcoholate or thiolate (as described above) in an aprotic solvent such as N,N-dimethylformamide, diglyme, ether or dimethylsulphoxide or in the alcohol or thiol corresponding to R and the reaction is allowed to proceed, at a temperature of 20°C to 150°C preferably up to 100°C, until the solution is no longer basic. The ω-halogenated ether or thioether of formula $R(CH_2)_nX$ may then be used to produce compounds of formula (I).

Compounds of formula (I) as hereinbefore defined are useful for treating coccidial infections, or preventing them, in livestock. The compounds may be administered alone, or in association with carriers. The present invention therefore also provides a compound of formula (I) for use in medicine; more particularly the invention provides a compound of formula (I) for use in the treatment or prevention of coccidiosis in livestock.

In a further aspect of the present invention there is provided a pharmaceutical composition comprising at least a compound of formula (I) for administration to livestock.

It may be convenient to administer the compounds in association with various carriers and additives to facilitate that administration. In particular, the compounds may be administered in the foodstuff or drinking water provided for the livestock.

The present invention, in a further aspect, therefore provides a pharmaceutical composition comprising a compound of formula (I) in association with a carrier therefor.

Carriers are materials which are useful for the purpose of administering the compound while being otherwise inert as regards interaction with the compound and non toxic to the recipient of the composition. It is particularly preferred that the carrier is the foodstuff or drinking water provided for the livestock.

When incorporated into foodstuff or drinking water the compounds may be administered at a concentration of about 10 ppm to 400 ppm preferably 50 ppm to 200 ppm and most preferably 100 ppm.

4-Cinnamylthio-1-β-D-ribofuranosylpyrazolo[3,4-*d*]pyrimidine has not been observed to cause any toxicity or reduction in weight gain in chicks when administered at a concentration of 800 ppm in the diet.

Some compounds of formula (I) are insufficiently soluble for administration in drinking water. In this case the phosphate ester, or more preferably, a salt thereof can be employed.

In a further aspect of the present invention there is provided a method for preventing or treating coccidial infections of livestock comprising the administration of an effective anticoccidial amount of a compound of formula (I) or a formulation or composition thereof.

The invention will now be illustrated by the following Examples, which should not be construed as limiting the invention in any way.

## Example 1

Preparation of 4-cinnamylthio-1-β-D-ribofuranosylpyrazolo(3,4-*d*)pyrimidine.

Cinnamyl bromide (3.04 g) was added to a stirred solution of 4-mercapto-1-β-D-ribofuranosylpyrazolo[3,4-*d*]pyrimidine (4.0 g) and potassium carbonate (2.13 g) in N,N-dimethylformamide. The solution was heated (40°C on an oil bath) for 2 hours. After cooling the reaction mixture was poured into water (0.6 l) and the resultant precipitate was collected and washed with water. The 4 - cinnamylthio - 1 - β - D - ribofuranosylpyrazolo[3,4-*d*]pyrimidine so obtained was recrystallised from methanol, washed with water and dried (*in vacuo* at 55°C), yield 3.2 g, m.p. 165—166°C.

## Example 2

Preparation of 4-mercapto-1-(2,3,5-tri-O-acetyl-β-D-ribofuranosyl)pyrazolo[3,4-*d*]pyrimidine.

4-Mercapto-1-β-D-ribofuranosylpyrazolo[3,4-d]pyrimidine (1.0 g) and potassium carbonate (0.48 g) were added to acetic anhydride (5 ml). The reaction was stirred at ambient temperature for 19 hours and heated at 70°C for 1.5 hour. Methanol was added and the whole was taken to dryness *in vacuo.* Water was added to the residue and it was filtered *in vacuo.* The aqueous filtrate was extracted 3 times with chloroform. The combined chloroform extracts were dried over magnesium sulfate, filtered and

taken to dryness *in vacuo*. The solid 4 - mercapto - 1 - (2,3,5 - tri - O - acetyl - β - D - ribofuranosyl)pyrazolo[3,4-*d*]pyrimidine (1.0 g, 70%), was collected and dried *in vacuo* at 70°C.

Analysis Calc'd for $C_{16}H_{18}N_4O_7S.\frac{1}{2}H_2O$

Theory: C: 45.82% H: 4.57% H: 13.36% S: 7.64%

Found: C: 45.85% H: 4.20% N: 13.20% S: 7.63%

Example 3

Preparation of 4-Cinnamylthio-1-(2,3,5-tri-O-acetyl-1-β-D-ribofuranosyl)pyrazolo[3,4-*d*]pyrimidine

A suspension of 4-mercapto-1-(2,3,5-tri-O-acetyl-β-D-ribofuranosyl)pyrazolo[3,4-*d*]pyrimidine (1.0 g) and potassium carbonate (0.37 g) in N,N-dimethylformamide was stirred for 10 minutes and then cinnamyl bromide (0.53 g) was added. After stirring for 3 hr. at 40°C the solution was filtered and 100 ml chloroform was added. The solution was extracted with water. The chloroform layer was dried, filtered and taken to dryness on a rotary evaporator. The residue was purified on a column of silica gel using chloroform as the eluant. The fractions containing the product were pooled and taken to dryness *in vacuo*. The residue was dissolved in chloroform, filtered through a Millipore filter *in vacuo* and evaporated *in vacuo*, to give the product 4 - cinnamylthio - 1 - (2,3,5 - tri - O - acetyl - β - D - ribofuranosyl)pyrazolo[3,4-*d*]pyrimidine as a glass.

Analysis Calc'd for $C_{25}H_{26}N_4O_7S$:—

Theory: C: 57.02% H: 4.98% N: 10.64% S: 6.09%

Found: C: 57.28% H: 4.92% N: 10.22% S: 5.85%

Example 4

Preparation of 4-Cinnamylthio-1-β-D-ribofuranosylpyrazolo[3,4-*d*]pyrimidine-5′-monophosphate disodium salt

The 4-cinnamylthio-1-β-ribofuranosylpyrazolo[3,4-*d*]pyrimidine of Example I (0.5 g) was added to triethyl phosphate (4 ml). The mixture was stirred and cooled in a stoppered flask (on —10°C bath). Phosphorus oxychloride (0.48 ml) was added all at once. The reaction was stirred (at —10°C) for 10 minutes and then at 0°C for 45 minutes. The reaction was then maintained at 0 to +5°C for 25 minutes more. The solution was poured onto ice and 2N sodium hydroxide was added to give pH 7. The solution was extracted with chloroform and then ether. The aqueous phase was adjusted to give pH 7.58.

Ether was removed *in vacuo* from the neutralized solution (at 30°C for 15 minutes). One-half of this solution was applied to a column containing Amberlite XAD-resin (200 ml) which had been equilibrated with water. The column was washed with three column volumes of water to elute sodium phosphate. The nucleotide was eluted with eight column volumes of ethanol in water (1:1).

The remaining half of the neutralized solution was treated similarly using a 100 ml column of resin.

Both nucleotide pools were combined and lyophilized. The lyophilized powder was dissolved in water (15 ml) and applied to a 5 × 100 cm column containing Bio Gel P—2. The nucleotide was eluted with water. Fractions containing the nucleotide were pooled and lyophilized. The powder was dissolved in water (5 ml) and precipitated by adding n-propanol (50 ml). This step was repeated and the final precipitate was lyophilized. The overall yield of 4 - cinnamyl - thio - 1 - β - D - ribofuranosylpyrazolo[3,4-*d*]pyrimidine - 5′ - monophosphate disodium salt, was 60% (0.36 g). Purity was estimated by high performance liquid chromatography to be 99%.

Example 5

Preparation of 4-(5-phenyl-2,4-pentadienylthio)-1-β-D-ribofuranosyl pyrazolo[3,4-*d*]pyrimidine

This compound was prepared in a manner exactly analogous to the method of Example 1, except that the reaction was conducted at ambient temperature for 20 hours. Yield of 4 - (5 - phenyl - 2,4 - pentadienylthio) - 1 - β - D - ribofuranosylpyrazolo[3,4-*d*]pyrimidine was 300 mg, m.p. 145—149°C.

Example 6

Preparation of 4-ethylthio-1-β-D-ribofuranosylpyrazolo[3,4-*d*]pyrimidine

Dowex (Registered Trade Mark) 1—X8 (bicarbonate) (4.0 g) was mixed with 4 - mercapto - 1 - β - D - ribofuranosylpyrazolo[3,4-*d*]pyrimidine (2.0 g) and methanol (0.1 l) was added. The mixture was warmed and stirred until no ultra-violet absorbing material remained in solution. Ethyl bromide (0.76 g) was added and the mixture stirred at ambient temperature for 20 hours. The resin was removed by vacuum filtration and washed with methanol. The filtrate and washings were combined

and the methanol evaporated *in vacuo* to afford a light tan-coloured solid. Yield of 4 - ethylthio - 1 - β - D - ribofuranosyl pyrazolo[3,4-*d*]pyrimidine 1.81 g, mp. 157—159°C.

Examples 7 to 10

The compounds of Examples 7 to 10 were prepared by a method exactly analogous to that of Example 6 using the appropriate alkyl halide, the duration of the reaction being varied as shown below.

| Example | Halide | Duration (days) | Product (1-β-D-ribofuranosyl-pyrazolo[3,4-*d*]pyrimidine | m.p. (°C) |
|---|---|---|---|---|
| 7 | I | 6 | 4-propylthio- | 93—95 |
| 8 | Br | I | 4-butylthio- | 88—89 |
| 9 | Br | 6 | 4-hexylthio- | 71—73 |
| 10(a) | I | 6 | 4-heptylthio- | 70—72 |
| 10(b) | Cl | 4.5 | 4-benzylthio- | 123—124.5 |

Example 11

Preparation of 4-pentylthio-1-β-D-ribofuranosyl pyrazolo[3,4-*d*]pyrimidine

4-Pentylthio-1-β-D-ribofuranosylpyrazolo[3,4-*d*]pyrimidine (m.p. 81—80°C) was prepared according to a method exactly analogous to the method of Example 6 except that the reaction with pentyl bromide, was performed at 60°C for 5 hours.

Example 12

Preparation of 4-pentylthio-1-β-D-ribofuranosyl pyrazolo[3,4-*d*]pyrimidine

4-mercapto-1-β-D-ribofuranosyl pyrazolo[3,4-*d*]pyrimidine (10 g) was added to ethanol (0.25 l) containing aqueous sodium bicarbonate (3.0 g in 0.025 l). Pentyl bromide solution (5.28 g) in ethanol, 0.01 l) was added dropwise with stirring. The mixture was allowed to stand for 1 hour at ambient temperature then heated to 50°C for 4 days. The reaction mixture was dried (*in vacuo*) and extracted into chloroform and water. The chloroform was separated and evaporated to a syrup (*in vacuo*) which was triturated with hexane and warm petroleum ether to afford a solid. The solid was dissolved in ethyl acetate, filtered and the filtrate was washed with water, then dried over sodium sulphate. After filtration the solution was evaporated to dryness (*in vacuo*) and the solid was collected. Yield 9.5 g of 4 - pentylthio - 1 - β - D - ribofuranosylpyrazole[3,4-*d*]pyrimidine (m.p. 79.5—82.5°C).

Example 13

Preparation of 4-ethylthio-1-β-D-ribofuranosylpyrazolo[3,4-*d*]pyrimidine-5'-monophosphate disodium salt

The compound of Example 6 (1.0 g) was added to triethyl phosphate (8 ml) and the mixture warmed to dissolve the compound. The mixture, in a stoppered flask, was cooled to —10°C on a bath and stirred. Phosphorus oxychloride (1.23 ml) was added in one portion and the mixture was maintained at —10°C for 40 minutes. The temperature was raised to —5°C for a further 30 minutes. The solution was poured onto ice and sodium hydroxide (2*M*) added to afford pH7. The mixture was extracted with chloroform (50 ml) and ether (50 ml) and further sodium hydroxide was added to pH 7.2.

The neutralised solution was diluted to 150 ml with cold water and kept on ice while acid-washed charcoal (65 ml) was added in portions until 99% of the nucleotide was absorbed from solution. The charcoal was washed twice with cold water. The nucleotide was eluted from the charcoal at 37°C using ethanol (95%)/water/ammonia solution (15 *M*) (5:4:1 by volume) (4 x 200 ml). Ethanol and ammonia were removed *in vacuo* at 37°C and the charcoal was filtered off using an Amicon pressure cell with PM—10 membrane. The nucleotide was converted to its sodium salt by passage through a column of Dowex 50—X8 resin (sodium form) (20 ml) yielding 1.1 g of 4 - ethylthio - 1 - β - D - ribofuranosyl-pyrazolo[3,4-*d*]pyrimidine-5'-monophosphate disodium salt. Purity was estimated to be 97% by high performance liquid chromatography.

Example 14 and 15

The compounds of Example 14 and 15 were prepared using a method exactly analogous to that used in Example 6 except that the duration of reaction was varied as shown.

| Example | Halide | Duration (days) | Product (1-β-D-ribofuranosyl-pyrazolo[3,4-*d*]pyrimidine | m.p. (°C) |
|---|---|---|---|---|
| 14 | Br | 3 | 4-(2-phenylethylthio)- | 85—87 |
| 15 | Br | 3 | 4-(3-phenylpropylthio)- | 95—97 |

Example 16 and 17

The compounds of Example 16 and 17 were prepared using a method exactly analogous to that used in Example 12 except that the reaction was conducted at reflux for the duration shown.

| Example | Halide | Duration (Hours) | Product (1-β-D-ribofuranosyl-pyrazolo[3,4-*d*]pyrimidine) | m.p. (°C) |
|---|---|---|---|---|
| 16 | Cl | 24 | 4-(3-(4-methylphenyl)-propylthio)$\frac{1}{2}H_2O$ | 188—120.5 |
| 17(a) | Cl | 41 | 4-(3-(4-chlorophenyl)-propylthio) | 129.5—130.5 |
| 17(b) | Cl | 48 | 4-(2-4-chlorophenyl)-ethylthio | 103—105 (softens 100) |

Example 18—20

The compounds of Example 18 to 20 were prepared by a method exactly analogous to that of Example 1 except that the reaction was conducted for the duration shown.

| Example | Halide | Duration (hours) | Product (1-β-D-ribofuransyl-pyrazolo[3,4-*d*]pyrimidine) | (°C) |
|---|---|---|---|---|
| 18 | Cl | 24 | 4-(2-phenoxyethylthio) | 125—126 |
| 19 | Cl | 24 | 4-(2-(4-methylphenyl)-ethylthio) | 96—97 |
| 20(a) | Cl | 24 | 4-(2-(3-methylphenyl) ethylthio) | 88—91 |
| 20(b) | Cl | 3 | 4-(4-benzyloxybenzyl-thio)- | 181—185 |
| 20(c) | CL | 20 | 4-(4-chlorocinnamyl-thio)- | 113—116 |
| 20(d) | Cl | 20 | 4-(3,4-dichloro-cinnamylthio)- | 62—67 |
| 20(e) | Cl | 4 | 4-(2,4-dichloro-cinnamylthio)- | 96 (softens 90) |
| 20(f) | Cl | 1 | 4-(3-phenyl-2-propynylthio) | 137 (softens 134) |
| 20(g) | Cl | 0.5 | 4-(3-trifluoromethyl-cinnamylthio) | 105—110 |

Example 21 to 23

The compounds of Example 21 to 23 were prepared using a method exactly analogous to that used in Example 12

| Example | Product (1-β-D-ribofuranoxyl pyrazolo(3,4-*d*]pyrimidine) | m.p. (°C) |
|---|---|---|
| 21 | 4-(4-methylbenzylthio)- | 125—127 |
| 22 | 4-(4-chlorobenzylthio)- | 114—117 (soften 80) |
| 23 | 4-(3-chlorobenzylthio)- | 90 (soften 75) |

Example 24

Preparation of 4-(5-phenylpentylthio)-1-β-D-ribofuranosylpyrazolo[3,4-*d*]pyrimidine

Crude 5-phenylpentyl chloride (1.3 g) was added to a stirred solution of 4 - mercapto - 1 - β - D - ribofuranosylpyrazolo[3,4-*d*]pyrimidine (2.0 g) and potassium bicarbonate (0.7 g) in N,N-dimethylformamide. The reaction mixture was heated on a steam bath for 25 hours. An additional 0.7 g of potassium bicarbonate was added and after heating for 1 hour more, the mixture was poured into water. The cooled aqueous mixture was extracted with chloroform. The chloroform soluble material was chromatographed on a silica gel column. The fractions containing the compound were combined and evaporated. Trituration with ether gave 0.4 g of crude product. This was dissolved in ethyl acetate and washed with water. The dried ethyl acetate solution was evaporated and purified by reversed phase chromatography in methanol water (80:20 vol/vol) to give 0.28 g of product m.p. 72—75°C (indefinite).

Analysis Calc'd for $C_{21}H_{26}N_4O_4S$

Theory: C: 58.58% H: 6.09% N: 13.01% S: 7.45%

Found: C: 58.83% H: 6.15% N: 13.06% S: 7.57%

Example 25

In order to assess the activity of compounds of formula (I) against coccidia, the compounds were administered to groups of 5 male Ross Ranger chicks (7 days old), at various dosages in the diet, for 6 days. The chicks were each infected with *Eimeria tenella* and *E. acervulina* one day after the beginning of the medication. The compounds had some effect on the *E. acervulina* and cleared chicks of *E. tenella* as indicated in Table 1 below. No obvious signs of toxicity were observed during this experiment.

TABLE 1.

Number of chicks cleared of *E. tenella* by administration of compounds of formula (I) at various dose levels.

| Compound of Example No. | Dose level (ppm of diet) 200 | 100 | 50 | 25 |
|---|---|---|---|---|
| 1 | 5 | 5 | 5 | 5 |
| 3 | 5 | NT | 5 | 1 |
| 4 | 5 | 5 | 5(5) | 5(4) |
| 5 | 5 | NT | 5 | 1 |
| 6 | 5 | 5 | 5 | 2 |
| 7 | 4 | 1 | 0 | 0 |
| 8 | 5 | 1 | 0 | 0 |
| 9 | 5 | 2 | 0 | 0 |
| 10 | 1 | NT | NT | NT |
| 11 | 5 | 3 | 0 | 0 |
| 13 | 5 | 4 | 4 | 0 |
| 14 | 5 | 1 | 1 | 1 |

| Compound of Example No. | Dose level (ppm of diet) 200 | 100 | 50 | 25 |
|---|---|---|---|---|
| 15 | 5 | 5 | 4 | 1 |
| 16 | 1 | NT | NT | NT |
| 17 | 5 | NT | 0 | 1 |
| 18 | 2 | NT | NT | NT |
| 19 | 2 | NT | NT | NT |
| 20(a) | NT | 1 | NT | NT |
| 20(b) | 5 | 5 | 5 | 3 |
| 20(c) | 5 | 5 | 0 | 0 |
| 20(d) | 2 | NT | NT | NT |
| 20(e) | 2 | NT | NT | NT |
| 20(f) | 5 | 2 | 0 | 1 |
| 20(g) | NT | NT | NT | NT |
| 21 | 5 | 5 | 4 | 2 |
| 22 | 5 | 5 | 4 | 3 |
| 23 | 1 | NT | NT | NT |

( ) indicates results of repeat experiment
NT not tested.

Example 26

Tolerance of chicks to 4-cinnamylthio-1-$\beta$-D-ribofuranosyl pyrazolo[3,4-*d*]pyrimidine

The compound of Example 1 was administered at various dosages to groups of 15 uninfected 7-day old male Ross Ranger chicks for 8 days. At the end of that period the weight gain of each group of chicks was recorded as a percentage of the weight gain of an untreated control group (Table II).

TABLE II

| Group | Treatment (ppm of diet) | Weight gain (%) |
|---|---|---|
| 1 | 100 | 102.2 |
| 2 | 50 | 110.5 |
| 3 | 25 | 108.4 |
| 4 | 12.5 | 107.4 |
| 5 | Untreated | 100 |

Example 27

The compounds of Examples 2 and 6 were tested *in vitro* for activity against coccidia of the species *Eimeria tenella*. Cell cultures were infected with sporozoite suspensions of *E. tenella* immediately after addition of the compounds. Serial dilutions of formulations containing the compounds were made in the range of 19 $\mu$g/l to 20 mg/l in order to determine the minimum active concentration. After incubation for 96 hours the cultures were fixed and the cells were stained with 0.1% toluidine blue. The stained cultures were examined microscopically for presence of parasites. The

compound of Example 1 cleared the cell cultures of *E. tenella* at all concentrations down to 78 μg/l and that of Example 6 cleared the cultures of concentrations down to 0.32 mg/l.

**Claims**

1. A compound of formula (I)

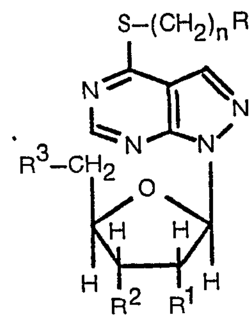

(I)

wherein n is an integer of 1 to 6 and R is a methyl, $(C_{1-4})$ alkoxy or $(C_{1-4})$alkylthio group or a phenoxy or phenylthio group or an unsubstituted or monosubstituted phenyl group, or when n has the value 1, a group $—(CH{=}CH)_mR^5$ wherein m has the value 1 or 2 or a group $—C{\equiv}C—R^5$ wherein $R^5$ is an unsubstituted or mono-, di-, or tri-substituted phenyl group, substituents for either of the aforementioned phenyl groups being selected from halogen atoms and $(C_{1-4})$alkyl, $(C_{1-4})$alkoxy, trifluoromethyl, benzyloxy, phenoxy, amino, mono- or di-$(C_{1-4})$alkylamino and hydroxyl groups, and either $R^1$, $R^2$ and $R^3$ are the same and are hydroxyl or acyloxy groups $—O—CO—R^4$ wherein $R^4$ is a hydrogen atom or a $(C_{1-4})$alkyl group or a substituted or unsubstituted phenyl group or $R^1$ and $R^2$ are hydroxyl or acyloxy groups as hereinbefore defined and $R^3$ is a phosphate group, or a salt thereof.

2. A compound as claimed in claim 1 wherein n has the value 1.

3. A compound as claimed in claim 1 or claim 2 wherein R is a methyl group of a group $—(CH{=}CH)_mR^5$.

4. A compound as claimed in any one of claims 1 to 3 wherein $R^1$ and $R^2$ are hydroxyl and $R^3$ is hydroxyl or a phosphate group or a salt thereof.

5. A process for producing compounds of formula (I) characterised in that it comprises the reaction between the precursor, a 4-(substituted) - 1 - β - D - ribofuranosylpyrazolo[3,4-*d*]-pyrimidine and a compound $R(CH_2)_nX$ wherein n and R are as hereinbefore defined and X is a halogen atom and the 4-substituent of the precursor is a thio group and the reaction is performed in the presence of an organic or inorganic base or a basic resin in an aqueous, a lower alcoholic or an aprotic solvent.

6. A process as claimed in claim 5 wherein the 4-substituent of the precursor is a mercapto group.

7. A compound of formula (I) for use in medicine.

8. A compound of formula (I) for use in the treatment or prevention of coccidiosis in livestock.

9. A pharmaceutical composition comprising a compound of formula (I) in association with a carrier therefor.

10. A composition as claimed in claim 9 wherein the carrier is a foodstuff or drinking water.

**Revendications**

1. Composé de formule (I)

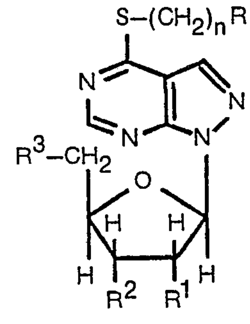

(I)

où n est un nombre entier de 1 à 6 et R est un radical méthyle, $(C_{1-4})$alcoxy ou $(C_{1-4})$alcoylthio ou un radical phénoxy ou phénylthio ou bien un radical phényle non substitué ou monosubstitué ou, lorsque n

a la valeur 1, un radical $-(CH{=}CH)_mR^5$ où m a la valeur 1 ou 2 ou un radical $-C{\equiv}C-R^5$ où $R^5$ est un radical phényle non substitué ou mono-, di- ou trisubstitué, les substituants pour l'un quelconque des radicaux phényle précités étant choisis entre les atomes d'halogène et radicaux $(C_{1-4})$alcoyle, $(C_{1-4})$alcoxy, trifluorométhyle, benzyloxy, phénoxy, amino, mono- ou di$(C_{1-4})$alcoylamino et hydroxyle et soit $R^1$, $R^2$ et $R^3$ sont identiques et sont des radicaux hydroxyle ou acyloxy $-O-CO-R^4$ où $R^4$ est un atome d'hydrogène ou un radical $(C_{1-4})$alcoyle ou un radical phényle substitué ou non substitué, soit $R^1$ et $R^2$ sont des radicaux hydroxyle ou acyloxy tels que définis ci-dessus et $R^3$ est un radical phosphate, ou un sel de celui-ci.

2. Composé suivant la revendication 1, dans lequel n a la valeur 1.

3. Composé suivant la revendication 1 ou 2, dans lequel R est un radical méthyle ou un radical $-(CH{=}CH)_mR^5$.

4. Composé suivant l'une quelconque des revendications 1 à 3, dans lequel $R^1$ et $R^2$ sont des radicaux hydroxyle et $R^3$ est un radical hydroxyle ou un radical phosphate, ou un sel de celui-ci.

5. Procédé de préparation des composés de formule (I) caractérisé en ce qu'il comprend la réaction entre le précurseur, une 1-$\beta$-D-ribofuranosylpyrazolo[3,4-d]pyrimidine 4-substituée et un composé $R(CH_2)_nX$ où n et R sont tels que définis ci-dessus, X est un atome d'halogène et le substituant en position 4 du précurseur est un radical thio, la réaction étant exécutée en présence d'une base organique ou inorganique ou d'une résine basique dans un solvant aqueux, un solvant alcoolique inférieur ou un solvant aprotique.

6. Procédé suivant la revendication 5, dans lequel le substituant en position 4 du précurseur est un radical mercapto.

7. Composé de formule (I) à utiliser en médecine.

8. Composé de formule (I) utilisé pour le traitement ou la prévention de la coccidiose chez les animaux d'élevage.

9. Composition pharmaceutique, comprenant un composé de formule (I) en association avec un excipient.

10. Composition suivant la revendication 9, dans laquelle l'excipient est un aliment ou de l'eau de boisson.

**Patentansprüche**

1. Verbindung der allgemeinen Formel I

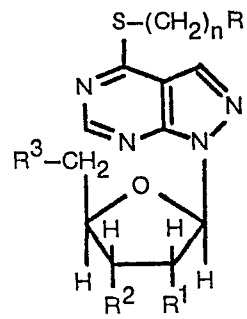

(I)

worin n eine ganze Zahl von 1 bis 6 bedeutet und R einen Methyl-, $(C_{1-4})$-Alkoxy- oder $(C_{1-4})$-Alkylthiorest oder einen Phenoxy- oder Phenylthiorest oder einen unsubstituierten oder monosubstituierten Phenylrest darstellt, oder wenn n den Wert 1 besitzt, einen Rest $-(CH{=}CH)_mR^5$ bedeutet, worin m den Wert 1 oder 2 hat oder einen Rest $-C{\equiv}C-R^5$, worin $R^5$ eine unsubstituierte oder mono-, di- oder trisubstituierte Phenylgruppe ist, wobei die Substituenten der jeweiligen zuvor genannten Phenylgruppen unter Halogenatomen und $(C_{1-4})$-Alkyl $(C_{1-4})$-Alkoxy, Trifluoromethyl, Benzyloxy, Phenoxy, Amino, Mono- oder Di-$(C_{1-4})$-alkylamino- und Hydroxylgruppen ausgewählt sind, und jedes $R^1$, $R^2$ und $R^3$ gleich ist und Hydroxylgruppen oder Acyloxygruppen $-O-CO-R^4$ bedeutet, worin $R^4$ ein Wasserstoffatom oder eine $(C_{1-4})$-Alkylgruppe oder eine substituierte oder unsubstituierte Phenylgruppe darstellt, oder $R^1$ und $R^2$ Hydroxyl- oder Acyloxygruppen gemäß der voranstehenden Definition sind, und $R^3$ eine Phosphatgruppe ist, und deren Salze.

2. Verbindung nach Anspruch 1, worin n den Wert 1 besitzt.

3. Verbindung nach Anspruch 1 oder 2, worin R eine Methylgruppe oder einen Rest $-(CH{=}CH)_mR^5$ bedeutet.

4. Verbindung nach einem der Ansprüche 1 bis 3, worin $R^1$ und $R^2$ Hydroxylgruppen sind und $R^3$ eine Hydroxylgruppe oder einen Phosphatrest bedeutet, oder ein entsprechendes Salz.

5. Verfahren zur Herstellung von Verbindungen der allgemeinen Formel I, dadurch gekennzeichnet, daß eine Umsetzung zwischen der Vorstufe, einem 4-(substituierten) - 1 - $\beta$ - D - Ribofuranosylpyrazolo[3,4-*d*] pyrimidin und einer Verbindung $R(CH_2)_nX$, worin n und R der zuvor angegebenen Definition entsprechen und X ein Halogenatom bedeutet und der 4-Substituent der Vorstufe

eine Thiogruppe ist, stattfindet, und die Reaktion in Gegenwart einer organischen oder anorganischen Base oder eines basischen Harzes in einem wäßrigen, einem Niederalkohol- oder einem aprotischen Lösungsmittel durchgeführt wird.

6. Verfahren nach Anspruch 5, worin der 4-Substituent der Vorstufe eine Mercaptogruppe ist.

7. Verbindung der Formel I zur Verwendung in der Medizin.

8. Verbindung der Formel I zur Verwendung in der Behandlung oder Verhütung von Coccidiosis in Tierhaltungen.

9. Pharmazeutische Zusammensetzung, enthaltend eine Verbindung der Formel I zusammen mit einem dafür geeigneten Träger.

10. Zusammensetzung nach Anspruch 9, worin der Träger ein Nahrungsmittel oder Trinkwasser ist.